# DEMANDE DE BREVET EUROPEEN

(11) **EP 1 486 183 A1**
(43) Date de publication de la demande: **15.12.2004**
(21) Numéro de dépôt: 03013026.4
(22) Date de dépôt: 10.06.2003
(51) Int. Cl.: A61F 2/38

(54) **Prothèse de genou**

(71) Demandeur: Medacta International S.A., 6874 Castel San Pietro (TI) (IT)
(72) Inventeur: Bellier, Guy, 75016 Paris (FR); Chassaing, Vincent, 92190 Meudon (FR); Combelles, Francois, 94120 Fontena Y Sous Bois (FR); LeFoll, Dominique, 95620 Parmain (FR); Sanouiller, Jean-Louis, 75006 Paris (FR); Staszewski, Jean-Michel, 91100 Villabe (FR); Bocquet, Francois, 62223 Sainte Catherine les Arras (FR)
(74) Mandataire: Incollingo, Italo

(57) **Abrégé**

La présente invention concerne les prothèses de genou implantables entre le fémur et le tibia d'un membre inférieur d'un être humain.

La prothèse selon l'invention comporte une pièce fémorale, un plateau tibial constitué de deux plaques et de moyens pour monter en rotation les deux plaques l'une par rapport à l'autre comportant deux faces de glissement 40, 41 réalisées respectivement sur les deux plaques, ces deux faces 40, 41 ayant une forme en "haricot" comportant chacune deux lobes respectivement externe 45, 46 et interne 47, 48, les deux lobes externe et interne de la seconde face 41 étant symétriques, les deux lobes internes 45, 46 respectivement des faces de glissement 40, 41 ayant sensiblement la même aire, tandis que le lobe interne 47 de la face de glissement 40 présente une aire supérieure à celle du lobe interne 48 de la face de glissement 41.

## Description

La présente invention concerne les prothèses de genou implantables entre le fémur et le tibia d'un membre inférieur d'un être humain, et plus particulièrement un perfectionnement à certaines prothèses de genou connues de l'art antérieur.

Il existe un type de prothèse de genou qui donne de bons résultats depuis de nombreuses années. Tel est le cas par exemple de la prothèse qui est décrite dans le brevet européen numéro 018 364.

La prothèse décrite dans ce document implantable entre le fémur et le tibia d'un membre inférieur d'un être humain, comporte une pièce fémorale apte à être fixée sur la tête distale du fémur, qui épouse sensiblement la forme d'un "U", le fond et l'un des côtés du "U" définissant deux surfaces condyliennes, l'autre côté du "U" définissant deux surfaces de trochlée séparées par une dépression définie selon une direction donnée

La prothèse comporte aussi un plateau tibial apte à coopérer avec la pièce fémorale et à être fixé sur la tête proximale du tibia. Ce plateau tibial est essentiellement constitué de deux plaques et de moyens pour monter en rotation ces deux plaques l'une par rapport à l'autre autour d'un axe de rotation sensiblement parallèle à l'axe du tibia. Une première plaque est apte à être fixée sur la tête proximale du tibia, la seconde plaque tournée vers la pièce fémorale est montée en coopération avec la pièce fémorale au moyen de deux surfaces de glissement congruentes des deux surfaces condyliennes.

Les moyens pour monter en rotation les deux plaques l'une par rapport à l'autre comportent deux faces de glissement réalisées respectivement sur les deux plaques, ces deux première et seconde faces de glissement étant sensiblement perpendiculaires à l'axe de rotation, un arbre de rotation solidaire en saillie de la seconde face de glissement suivant un axe sensiblement perpendiculaire à cette seconde face de glissement, un palier en creux réalisé dans la première plaque et débouchant sur la première face de glissement de façon que l'arbre de rotation puisse y pivoter autour de son axe. Les deux faces de glissement affectent sensiblement une forme de "haricot" et comportent de ce fait chacune deux lobes respectivement externe et interne, les deux lobes externe et interne de la seconde face de glissement étant sensiblement symétriques par rapport à une droite perpendiculaire à l'axe de rotation.

Comme mentionné ci-dessus, cette prothèse a donné de bons résultats alliant tout à la fois une bonne fiabilité, une structure relativement simple et une relative facilité d'implantation.

La présente invention a cependant pour but d'améliorer la fiabilité de cette prothèse, tout en lui conservant ses principaux avantages comme ceux mentionnés ci-dessus.

Plus précisément, la présente invention a pour objet une prothèse de genou implantable entre le fémur et le tibia d'un membre inférieur d'un être humain, comportant:
- une pièce fémorale apte à être fixée sur la tête distale du fémur, la pièce fémorale épousant sensiblement la forme d'un "U", le fond et l'un des côtés du "U" définissant deux surfaces condyliennes, l'autre côté du "U" définissant deux surfaces de trochlée séparées par une dépression définie selon une direction donnée,
- un plateau tibial apte à être fixé sur la tête proximale du tibia, le plateau tibial étant constitué de deux plaques et de moyens pour monter en rotation les deux dites plaques l'une par rapport à l'autre autour d'un axe de rotation sensiblement parallèle à l'axe du tibia, une première plaque étant apte à être fixée sur la tête proximale du tibia, la seconde plaque tournée vers la pièce fémorale étant montée en coopération avec la pièce fémorale au moyen de deux surfaces de glissement congruentes des deux surfaces condyliennes,
- lesdits moyens pour monter en rotation les deux dites plaques l'une par rapport à l'autre comportant deux première et seconde faces de glissement réalisées respectivement sur les deux première et seconde plaques, ces deux première et seconde faces de glissement étant sensiblement perpendiculaires audit axe de rotation, un arbre de rotation solidaire en saillie de la seconde face de glissement suivant un axe sensiblement perpendiculaire à cette seconde face de glissement, un palier réalisé dans la première plaque et débouchant sur la première face de glissement de façon que l'arbre de rotation puisse pivoter dans le palier autour de son axe, les deux première et seconde faces de glissement affectant chacune une forme en "haricot" et comportant de ce fait chacune deux lobes respectivement externe et interne, les deux lobes externe et interne de la seconde face de glissement étant sensiblement symétriques par rapport a une droite perpendiculaire audit axe de rotation,
caractérisée par le fait que les deux lobes externes respectivement des première et seconde faces de glissement ont sensiblement la même aire, tandis que le lobe interne de la première face de glissement présente une aire supérieure à celle du lobe interne de la seconde face de glissement de façon que, lorsque la seconde plaque pivote par rapport à la première plaque d'un angle donné α autour de l'axe de rotation, le lobe interne de la seconde face de glissement soit toujours complètement en appui sur le lobe interne de la première face de glissement.

D'autres caractéristiques et avantages de l'invention apparaîtront au cours de la description suivante donnée en regard des dessins annexés à titre illustratif mais nullement limitatif, dans lesquels :
La figure 1 représente les os du membre inférieur droit d'un être humain qui interviennent lors de l'implantation d'une prothèse de genou, et leur position respective.
Les figures 2 et 3 représentent respectivement en vue de côté et de face, un mode de réalisation d'une prothèse de genou selon l'invention pour une jambe gauche, comportant en association une pièce fémorale et un plateau tibial,
La figure 4 représente, en vue de dessus, les deux faces qui coopèrent par glissement l'une sur l'autre et qui appartiennent aux deux éléments constitutifs du plateau tibial d'une prothèse de genou selon l'invention pour une jambe droite,
Les figures 5 et 6 représentent deux vues en perspective, respectivement en éclaté et en coupe, d'un mode de réalisation de la pièce rotulienne de la prothèse de genou selon l'invention, et
La figure 7 représente, en vue de côté, une prothèse de genou complète selon l'invention implantée entre un fémur et un tibia, y compris l'ensemble des muscles du quadriceps et le tendon rotulien.

Il est bien précisé que, sur les figures, les mêmes références désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

Les Demandeurs tiennent aussi à préciser que les figures représentent essentiellement un seul mode de réalisation de l'objet selon l'invention et qu'il peut exister d'autres modes de réalisation qui répondent à la définition de cette invention.

Ils précisent en outre que, lorsque, selon la définition de l'invention, l'objet de l'invention comporte "au moins un" élément ayant une fonction donnée, le mode de réalisation décrit peut comporter plusieurs de ces éléments.

Ils précisent aussi que, si le mode de réalisation de l'objet selon l'invention tel qu'illustré comporte plusieurs éléments de fonction identique et que si, dans la description, il n'est pas spécifié que l'objet selon cette invention doit obligatoirement comporter un nombre particulier de ces éléments, l'objet de l'invention pourra être défini comme comportant "au moins un" de ces éléments.

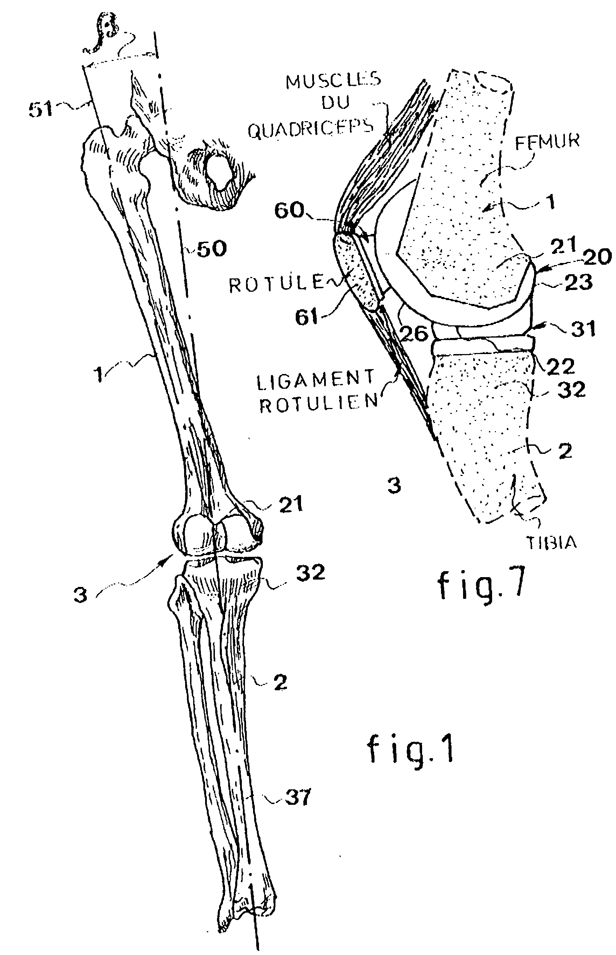

Ceci ayant été précisé, la présente invention concerne une prothèse de genou apte à être implantée entre le fémur 1 et le tibia 2 d'un membre inférieur 3 d'un être humain. La figure 1 représente, en vue de face, ces deux os d'une jambe droite dans leurs forme et position relative originelle avant l'implantation d'une prothèse de genou comme illustré sur la figure 7 en vue de côté, la structure osseuse de la jambe gauche étant symétrique de celle de la jambe droite.

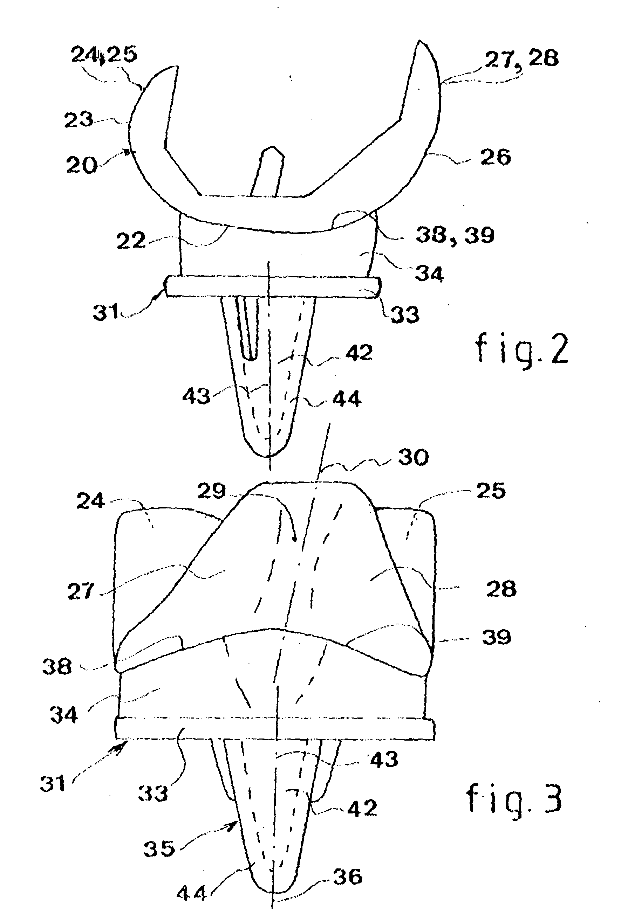

La prothèse de genou conforme à l'invention comporte une pièce fémorale 20 apte à être fixée sur la tête distale 21 du fémur Cette pièce fémorale épouse sensiblement la forme d'un "U", le fond 22 et l'un 23 des côtés du "U" définissant deux surfaces condyliennes 24, 25, l'autre côté 26 du "U" définissant deux surfaces de trochlée 27, 28 séparées par une dépression 29 définie selon une direction donnée 30, comme plus particulièrement illustré sur les figures 2 et 3.

Elle comporte aussi un plateau tibial 31 apte à être monté en coopération avec la pièce fémorale 20 et à être fixé sur la tête proximale 32 du tibia 2. Ce plateau tibial, comme plus particulièrement illustré sur les figures 2 et 3, est constitué de deux plaques 33, 34 et de moyens 35 pour monter en rotation ces deux plaques l'une par rapport à l'autre autour d'un axe de rotation 36 sensiblement parallèle à l'axe 37 du tibia (figure 1).

Une première plaque 33 est apte à être fixée sur la tête proximale du tibia, la seconde plaque 34 tournée vers la pièce fémorale 20 est, elle, apte à être montée en coopération avec la pièce fémorale au moyen de deux surfaces de glissement 38, 39 congruentes respectivement des deux surfaces condyliennes 24,25.

Les moyens 35 pour monter en rotation les deux plaques 33, 34 l'une par rapport à l'autre comportent deux première et seconde faces de glissement 40, 41 réalisées respectivement sur les deux première et seconde plaques 33, 34, ces deux première et seconde faces de glissement 40, 41 étant sensiblement perpendiculaires à l'axe de rotation 36, un arbre de rotation 42 solidaire en saillie de la seconde face de glissement 41 suivant un axe 43 sensiblement perpendiculaire à cette seconde face de glissement, un palier 44 réalisé dans la première plaque 33 et débouchant sur la première face de glissement 40 de façon que l'arbre de rotation 42 puisse pivoter dans ce palier 44 autour de son axe 43. L'arbre de rotation 42 et le palier 44 sont connus en eux-mêmes et n'ont donc été illustrés que très schématiquement en traits interrompus sur les figures 2 et 3.

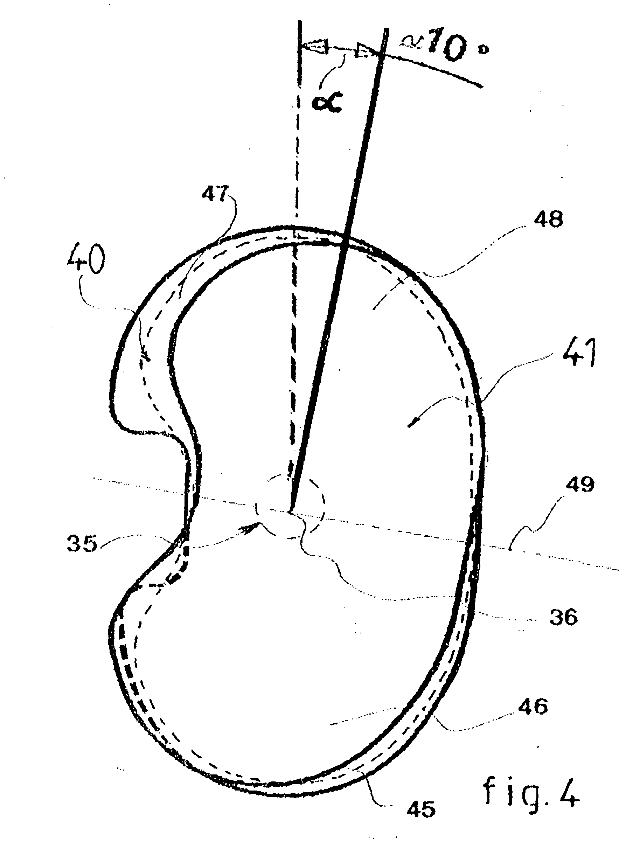

Les deux première et seconde faces de glissement affectent chacune une forme en "haricot", figure 4, et comportent de ce fait chacune deux lobes respectivement externe 45, 46 et interne 47, 48, les deux lobes externe 46 et interne 48 de la seconde face de glissement 41 étant sensiblement symétriques par rapport à une droite 49 perpendiculaire à l'axe de rotation 30.

Selon une caractéristique importante de l'invention, les deux lobes externes 45, 46 respectivement des première et seconde faces de glissement 40, 41 ont sensiblement la même aire, tandis que le lobe interne 47 de la première face de glissement 40 présente une aire supérieure à celle du lobe interne 48 de la seconde face de glissement 41 de façon que, lorsque la seconde plaque 34 pivote par rapport à la première plaque 33 d'un angle donné α autour de l'axe de rotation 36, figure 4, le lobe interne 48 de la seconde face de glissement 41 soit toujours complètement en appui sur le lobe interne 47 de la première face de glissement 40. Les deux positions limites de la face de glissement 41 par rapport à la face de glissement 40 sont respectivement représentées en traits continus et en traits interrompus sur cette figure 4, permettant de mettre en évidence que le lobe interne 48 de la seconde face de glissement de la seconde plaque 34 repose toujours sur le lobe interne 47 de la première face de glissement 40 de la première plaque 33.

De préférence, la valeur de cet angle donne α est au maximum de l'ordre de dix degrés, et peut être par exemple de l'ordre de cinq degrés pour certains cas de prothèses de genou

De façon très avantageuse, la direction donnée 30 définie ci-dessus, figure 3, fait avec la perpendiculaire aux première et seconde faces de glissement 40, 41, un angle sensiblement égal à l'angle "β", figure 1, que font entre eux l'axe mécanique 50 du fémur 1 qui passe par la tête du col du fémur et qui est sensiblement confondu avec l'axe 37 du tibia et perpendiculaire aux deux faces de glissement 40, 41, et son axe anatomique 51 qui est défini par la direction de la force de traction exercée par l'ensemble des muscles quadriceps notamment sur la rotule avec, pour force de réaction, celle exercée par le tendon rotulien, figure 7. La valeur de cet angle β est de l'ordre au mieux de huit degrés.

Dans certains cas, la rotule originelle est conservée. Cependant, dans la majorité des cas, il est fait ablation des surfaces de glissement complémentaires des surfaces de trochlée et, sur la portion d'os restante 61 de la rotule originelle, est implantée une pièce rotulienne, voir plus particulièrement la figure 7.

La prothèse de genou comporte donc aussi une pièce rotulienne 60 apte à être implantée dans la portion d'os restante 61 de la rotule originelle, cette pièce rotulienne étant apte à glisser sur les deux surfaces de trochlée 27, 28 de la pièce fémorale au moyen de deux parties de surface de glissement 62, 63 congruentes respectivement des deux surfaces de trochlée.

Dans une réalisation préférentielle, la pièce rotulienne 60 comporte, par référence aux figures 3, 6 et 7, un élément de vissage 64 comportant un embout fileté 65, par exemple de type osseux auto-taraudant, une tête d'épaulement 66 solidaire de cet embout fileté, et une plaque de support 67 comportant un orifice traversant 68 d'une section au moins égale à celle de l'embout fileté mais inférieure à celle de la tête d'épaulement

Elle comporte en outre un insert 69 comportant deux surfaces opposées 70, 71 comprenant une première surface dite "d'appui" 70 apte à venir au contact par glissement d'une surface de rotation 72 réalisée sur la plaque de support 67 et une seconde surface 71 comportant les deux parties de surface de glissement 62, 63 congruentes respectivement des deux surfaces de trochlée 27, 28, et des moyens 73 pour lier en rotation l'insert 69 avec l'élément de vissage 64 de façon en outre à emprisonner la tête d'épaulement 66 entre la plaque support 67 et l'insert 69.

Dans une réalisation avantageuse pour améliorer son implantation dans la portion d'os restante 61 de la rotule, l'embout fileté 65 comporte une percée axiale 74 débouchant par une ouverture 75 à son extrémité 76 opposée à celle 77 qui est solidaire de la tête d'épaulement 66.

En outre, dans le même but que défini ci-dessus, la paroi 78 sensiblement cylindrique de révolution de la percée axiale 74 comporte une pluralité d'orifices traversants 79, pour permettre à l'os se développant par ostéosynthèse de bien emprisonner l'embout fileté 65.

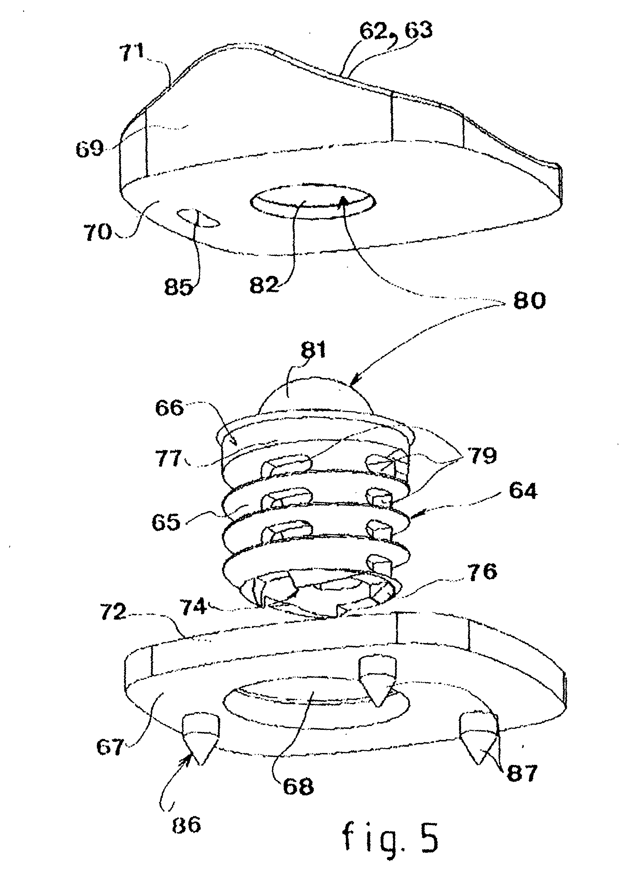

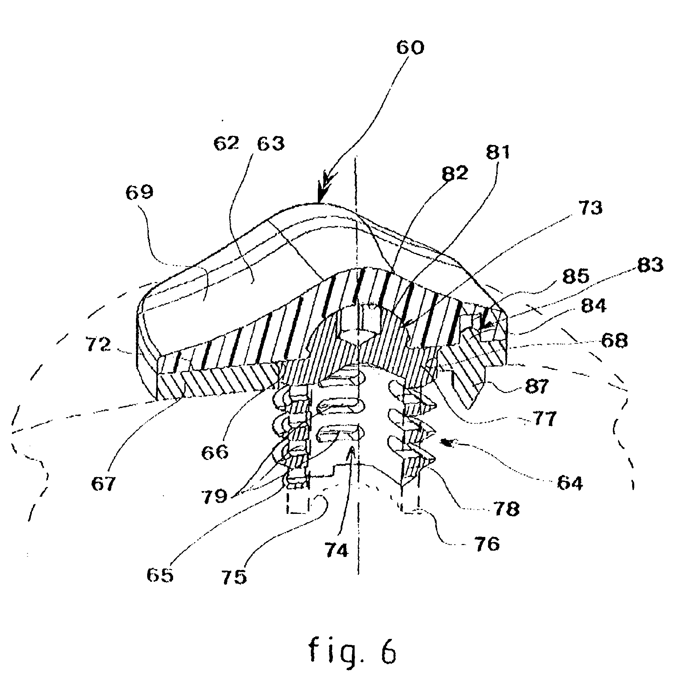

Dans la réalisation illustrée sur les figures 5 et 6, les moyens 73 définis ci-dessus pour lier en rotation l'insert 69 avec l'élément de vissage 64 de façon à emprisonner la tête d'épaulement 66 entre la plaque support 67 et l'insert 69, sont constitués par des moyens d'emboîtement enclipsables 80 constitués de deux parties essentiellement de révolution complémentaires mâle et femelle 81, 82, l'une des deux parties de révolution complémentaires étant solidaire de la tête d'épaulement 66, l'autre étant solidaire de l'insert 69, de façon à permettre à l'insert 69 de pivoter par rapport à la plaque de support 67, dans le but qui sera défini ci après

De plus et très avantageusement, la prothèse comporte en outre, pour la sécurité de son fonctionnement, des moyens 83 pour limiter la rotation de l'insert 69 par rapport à la plaque support 67. Ces moyens 83 comportent par exemple un ergot 84 solidaire de l'un des deux éléments "plaque support" et "insert", et une rainure 85 d'une longueur déterminée pour définir la rotation maximale de l'insert par rapport à la plaque support 67, réalisée dans l'autre de ces deux éléments, l'ergot et la rainure étant agencés respectivement sur ces deux éléments de façon que, lorsque les deux parties de révolution complémentaires mâle femelle sont enclipsées l'une dans l'autre, l'ergot plonge au moins partiellement dans la rainure et puisse s'y déplacer sur une certaine longueur courbe centrée sur l'axe de l'embout fileté 65

II est possible que la prothèse comporte en outre des moyens 86 pour fixer la position de la plaque support par rapport à la portion d'os restante de la rotule. Ces moyens 86 comportent par exemple au moins un picot 87, et de préférence trois, apte à s'implanter dans la portion d'os restante 61 de la rotule originelle.

Il est en outre précisé que l'ensemble des éléments de la prothèse selon l'invention est réalisé en métal comme du chrome cobalt, à l'exception de la seconde plaque 34 et de l'insert 69 qui sont tous deux en une matière comme du polyéthylène ou analogue, toutes ces matériaux étant du type biocompatible.

La prothèse du genou telle que décrite ci-dessus présente des avantages par rapport à cette de l'art antérieur comme celle définie dans le document référencé au préambule de la présente description.

Il est tout d'abord précisé que, lorsque la jambe se plie, il se produit généralement une double rotation autour de deux axes qui font entre eux un angle très proche d'un angle droit, une première rotation qui est de l'ordre de cent quarante degrés et une seconde rotation qui est de l'ordre de dix degrés.

Avec la prothèse selon l'invention, la seconde rotation est obtenue par le glissement en rotation des première et seconde faces de glissement. Comme la majorité des efforts s'exerce sur les lobes internes de ces deux faces de glissement, du fait de la structure de la prothèse selon l'invention, le lobe interne 48 de la seconde face de glissement demeure toujours en appui sur le lobe interne 47 de la première face de glissement. En conséquence, au contraire de la prothèse selon l'art antérieur définie ci-avant, tous les efforts sont entièrement constamment répartis sur le lobe interne 48 de la seconde face de glissement. En outre, le lobe externe 46 de la seconde face de glissement peut déborder du lobe externe de la première face sans que celui-ci subisse un endommagement consécutif à ce débordement, puisque tous les efforts sont reportés sur les lobes internes.

De plus, du fait de l'orientation de la dépression 29 entre les deux surfaces de trochlée 27, 28 suivant une direction sensiblement confondue avec celle de l'axe anatomique 51 du fémur, la plaque support 67 ne subit qu'une très faible rotation par rapport à l'insert 69 qui se déplace en glissement le long de cette dépression, ce qui réduit ainsi la possibilité d'une usure accélérée entre la plaque support et l'insert.

La structure de la prothèse de genou selon l'invention telle que décrite ci-dessus, permet de conserver tous les avantages de la prothèse similaire de l'art antérieur, tout en réduisant son encombrement et en lui conférant une plus grande fiabilité.

## Revendications

1. Prothèse de genou implantable entre le fémur (1) et le tibia (2) d'un membre inférieur (3) d'un être humain, comportant:
• une pièce fémorale (20) apte à être fixée sur la tête distale (21) du fémur, la pièce fémorale épousant sensiblement la forme d'un "U", le fond (22) et l'un (23) des côtés du "U" définissant deux surfaces condyliennes (24, 25), l'autre côté (26) du "U" définissant deux surfaces de trochlée (27, 28) séparées par une dépression (29) définie selon une direction donnée (30),
• un plateau tibial (31) apte à être fixé sur la tête proximale (32) du tibia (2), le plateau tibial étant constitué par deux plaques (33, 34) et des moyens (35) pour monter en rotation les deux dites plaques l'une par rapport à l'autre autour d'un axe de rotation (36) sensiblement parallèle à l'axe (37) du tibia, une première plaque (33) étant apte à être fixée sur la tête proximale du tibia, la seconde plaque (34) tournée vers la pièce fémorale (20) étant montée en coopération avec la pièce fémorale au moyen de deux surfaces de glissement (38, 39) congruentes des deux surfaces condyliennes (24, 25),
• lesdits moyens (35) pour monter en rotation les deux dites plaques l'une par rapport à l'autre comportant deux première et seconde faces de glissement (40, 41) réalisées respectivement sur les deux première et seconde plaques (33, 34), ces deux première et seconde faces de glissement (40, 41) étant sensiblement perpendiculaires audit axe de rotation (36), un arbre de rotation (42) solidaire en saillie de la seconde face de glissement (41) suivant un axe (43) sensiblement perpendiculaire à cette seconde face de glissement, un palier (44) réalisé dans la première plaque (33) et débouchant sur la première face de glissement (40) de façon que l'arbre de rotation (42) puisse pivoter dans le palier (44) autour de son axe (43), les deux première et seconde faces de glissement affectant chacune une forme en "haricot" et composant de ce fait chacune deux lobes respectivement externe (45, 46) et interne (47, 48), les deux lobes externe (46) et interne (48) de la seconde face de glissement (41) étant sensiblement symétriques par rapport à une droite (49) perpendiculaire audit axe de rotation (36).
**caractérisé par le fait que** les deux lobes externes (45, 46) respectivement des première et seconde faces de qlissement (40, 41) ont sensiblement la même aire, tandis que le lobe interne (47) de la première face de glissement (40) présente une aire supérieure à cette du lobe interne (48) de la seconde face de glissement (41) de façon que, lorsque la seconde plaque (34) pivote par rapport à la première plaque (33) d'un angle donné α autour de l'axe de rotation (36), le lobe interne (48) de la seconde face de glissement (41) soit toujours complètement en appui sur le lobe interne (47) de la première face de glissement (40).

2. Prothèse de genou selon la revendication 1, **caractérisée par le fait que** ladite direction donnée (30) fait, avec la perpendiculaire aux première et seconde faces de glissement (40, 41), un angle sensiblement égal à l'angle "β" que font entre eux l'axe mécanique (50) du fémur (1) et son axe anatomique (51).

3. Prothèse de genou selon la revendication 2, **caractérisée par le fait qu'**elle comporte en outre une pièce rotulienne (60) apte à être implantée dans une portion d'os (61) de la rotule originelle, ladite pièce rotulienne étant apte à glisser sur les deux surfaces de trochlée (27, 28) au moyen de deux parties de surface de glissement (62, 63) congruentes des deux surfaces de trochlée.

4. Prothèse de genou selon la revendication 3, **caractérisée par le fait que** ladite pièce rotulienne (60) comporte :
• un élément de vissage (64) comportant un embout fileté (65) et une tête d'épaulement (66) solidaire dudit embout fileté,
• une plaque de support (67), ladite plaque de support comportant un orifice traversant (68) d'une section au moins égale à celle dudit embout fileté mais inférieure à celle de ladite tête d'épaulement,
• un insert (69) comportant deux surfaces opposées (70, 71) comprenant une première surface dite "d'appui" (70) apte à venir au contact par glissement d'une surface de rotation (72) réalisée sur ladite plaque de support (67) et une seconde surface (71) comportant les deux dites parties de surface de glissement (62, 63) congruentes des deux surfaces de trochlée (27, 28), et
• des moyens (73) pour lier en rotation ledit insert (69) avec l'élément de vissage (64) de façon en outre à emprisonner ladite tête d'épaulement (66) entre la plaque support (67) et ledit insert (69).

5. Prothèse de genou selon la revendication 4, **caractérisée par le fait que** ledit embout fileté (65) comporte une percée axiale (74) débouchant par une ouverture (75) à son extrémité (76) opposée à cette (77) qui est solidaire de la tête d'épaulement (66).

6. Prothèse de genou selon la revendication 5, **caractérisée par le fait que** la paroi (78) sensiblement cylindrique de révolution de ladite percée axiale (74) comporte une pluralité d'orifices traversants (79).

7. Prothèse du genou selon l'une des revendications 4 à 6, **caractérisée par le fait que** les moyens (73) pour lier en rotation ledit insert (69) avec l'élément de vissage (64) de façon à emprisonner ladite tête d'épaulement (66) entre la plaque support (67) et ledit insert (69), sont constitués par des moyens d'emboîtement enclipsables (80) constitués de deux parties de révolution complémentaires mâle et femelle (81, 82), l'une des deux parties de révolution complémentaires étant solidaire de la tête d'épaulement (66), l'autre étant solidaire de l'insert (69).

8. Prothèse de genou selon la revendication 7, **caractérisée par le fait qu'**elle comporte des moyens (83) pour limiter la rotation dudit insert (69) par rapport ladite plaque support (67).

9. Prothèse de genou selon la revendication 8, **caractérisée par le fait que** les moyens (83) pour limiter la rotation dudit insert par rapport à ladite plaque support comportent un ergot (84) solidaire de l'un des deux éléments "plaque support" et "insert", et une rainure (85) d'une longueur déterminée réalisée dans l'autre de ces deux éléments, ledit ergot et ladite rainure étant agencés respectivement sur ces deux éléments de façon que, lorsque les deux parties de révolution complémentaires mâle-femelle sont enclipsées l'une dans l'autre, ledit ergot plonge au moins partiellement dans ladite rainure.

10. Prothèse de genou selon l'une des revendications 4 à 9, **caractérisée par le fait qu'**elle comporte des moyens (86) pour fixer la position de ladite plaque support par rapport à ladite portion d'os de la rotule.

11. Prothèse de genou selon la revendication 8, **caractérisée par le fait que** los moyens (86) pour fixer la position de ladite plaque support par rapport à ladite portion d'os de la rotule comportent au moins un picot (87) apte à s'implanter dans ladite portion d'os (61) de la rotule originelle.

12. Prothèse de genou selon l'une des revendications 1 à 11, **caractérisée par le fait que** la valeur dudit angle donné α est au maximum de l'ordre de dix degrés.

13. Prothèse de genou selon l'une des revendications 1 à 11, **caractérisée par le fait que** la valeur dudit angle β est de l'ordre de huit degrés.
